# EUROPEAN PATENT SPECIFICATION

(11) **EP 3 537 988 B1**
(45) Date of publication and mention of the grant of the patent: **02.09.2020**
(21) Application number: 17809195.5
(22) Date of filing: 07.11.2017
(51) Int. Cl.: A61B 17/00

(54) **A LIQUID DISPENSER**
FLÜSSIGKEITSSPENDER
DISTRIBUTEUR DE LIQUIDE

(30) Priority: 08.11.2016 EP 16197839
(43) Date of publication of application: 18.09.2019
(73) Proprietor: Connexicon Medical Limited, 24 Dublin (IE)
(72) Inventor: LEDWIDGE, Eadaoin D., Killiney, Co Dublin (IE); O BRENNAN, Martin, Dublin 18 (IE); LENNON, Oisin D, Dublin 7 (IE); MCHUGH, Niall F., Blessington, County Wicklow (IE)
(74) Representative: FRKelly
(86) International application number: PCT/EP2017/078482
(87) International publication number: WO 2018/087091

(56) References cited:
- WO-A1-2013/144849
- US-A1- 2002 076 255
- US-A1- 2006 049 203
- US-B1- 6 371 675

## Description

### Field of the invention

The present invention is concerned with a liquid dispenser, in particular for use as a medical device that can express a liquid in a controlled manner, such as a cyanoacrylate based adhesive, for example for topical application to close wounds in a surgical setting.

### Background of the invention

Wound closure in surgical applications is conventionally achieved using sutures, staples, or similar mechanical closures, although these systems do suffer from a number of drawbacks. Thus use of sutures or staples can be both painful during the surgical procedure, and will often result in scaring such as puckering where the tissue is pierced by the closure and where the edges of the wound are drawn tightly together.

As an alternative to the above mechanical type closure systems it is also known to use a liquid adhesive to effect closure of a wound, for example a cyanoacrylate based adhesive. Such adhesives are conventionally dispensed from a suitable device directly onto the wound to be closed, the surgeon drawing the opposed edges of the wound into apposition before applying the adhesive along the wound, the adhesive being designed to cure on contact with the skin. The adhesive may require the action of a catalyst such as an initiator in order to effect curing of the adhesive, for example through the action of polymerization. Alternative adhesives may however not require the use of such a catalyst.

The adhesive is conventionally supplied in a closed container such as a glass ampoule, which is then broken in order to allow the adhesive to be dispensed by a suitable dispensing device into which the ampoule may be located. Breaking of the glass ampoule can however give rise to problems, in particular the safe management of the broken glass.

US2006049203 discloses an applicator/dispenser for dispensing, mixing and/or applying a polymerizable monomeric adhesive or sealant material comprises: a body portion; an actuator movable relative to the body portion; a cavity in the body portion; and a piercing or breaking portion on the actuator. US2002076255 discloses applicator for an anti-microbial prep solution of this invention includes a generally hollow handle having a closed proximal end and an open distal end, a foam pad attached to the hollow handle over the open distal end, an ampoule that holds the anti-microbial solution therein and a means for opening the ampoule. US6371675 discloses an applicator for an anti-microbial prep solution of this invention includes a generally hollow handle having a closed proximal end and an open distal end, a foam pad attached to the hollow handle over the open distal end, and a slit formed in the foam pad that acts as a flow control valve.

It is an object of the present invention to provide an improved liquid dispensing device which is capable of effectively dispensing liquid from such an ampoule while also safely managing the broken glass resulting from breaching the ampoule to access the liquid.

It is a further object of the present invention to provide such an improved liquid dispensing device which is designed to be actuated with relatively little force in order to facilitate ease of operation, which is particularly important in a surgical setting.

### Summary of the invention

According to the present invention there is provided a liquid dispenser according to claim 1.

Preferably, the housing comprises one or more elongate ribs extending longitudinally of the chamber.

Preferably, the section of the housing permitting access to the chamber is defined by a window in the housing.

Preferably, the actuator comprises an abutment shaped and dimension to pass through the window.

Preferably, the actuator comprises a cantilever arm projecting from the housing.

Preferably, the abutment is located at or adjacent to a free end of the cantilever arm.

Preferably, the actuator is reversibly displaceable relative to the housing.

Preferably, the actuator is hingedly mounted to the housing.

Preferably, the actuator is biased away from the housing.

Preferably, at least a portion of the actuator is formed from a resiliently deformable material.

Preferably, the outlet comprises a nozzle in fluid communication with the chamber.

Preferably, the nozzle is releasably retained on the housing in order to permit access to the chamber.

Preferably, the housing comprises a substantially cylindrical hollow body.

Preferably, the housing is rigid.

Preferably, the liquid dispenser comprises an ampoule of a liquid shaped and dimensioned to be received in the chamber.

Preferably, the ampoule has a circular cross sectional area.

Preferably, the ampoule comprises a breachable wall.

Preferably, the breachable wall is at least partially comprised of glass or brittle plastic.

Preferably, the liquid dispenser comprises an outer sleeve locatable to surround the ampoule and having an outlet for the liquid.

Preferably, the outer sleeve is deformable.

Preferably, the liquid dispenser comprises a filter disposed between the chamber and the outlet.

Preferably, the liquid dispenser comprises an initiator operable, on contact with the liquid, to alter the physical and/or chemical characteristics of the liquid.

Preferably, the housing is at least partially transparent in order to provide visibility of the contents of the chamber.

Preferably, the housing may be shaped and dimensioned to provide a desired volume to the chamber.

Preferably, the liquid dispenser comprises a cartridge comprising the ampoule and the outer sleeve and optionally the filter.

Preferably, the cartridge is shaped and dimensioned to be received in the chamber.

Preferably, the cartridge has a circular cross section.

### Brief description of the drawings

The present invention will now be described with reference to the accompanying drawings, in which:
Figure 1 illustrates a perspective view of a liquid dispenser according to a preferred embodiment of the present invention;
Figure 2a illustrates a side elevation of the liquid dispenser;
Figure 2b illustrates an end elevation of the liquid dispenser;
Figure 3 illustrates an exploded side view of the liquid dispenser;
Figure 4 illustrates the arrangement of Figure 3 with a cartridge shown exploded to illustrate an ampoule and filter contained therein;
Figure 5 illustrates an exploded perspective view of the liquid dispenser;
Figure 6 illustrates a rear perspective view of a dispensing tip forming part of the liquid dispenser;
Figure 7 illustrates an alternative rear perspective view of the tip of Figure 6;

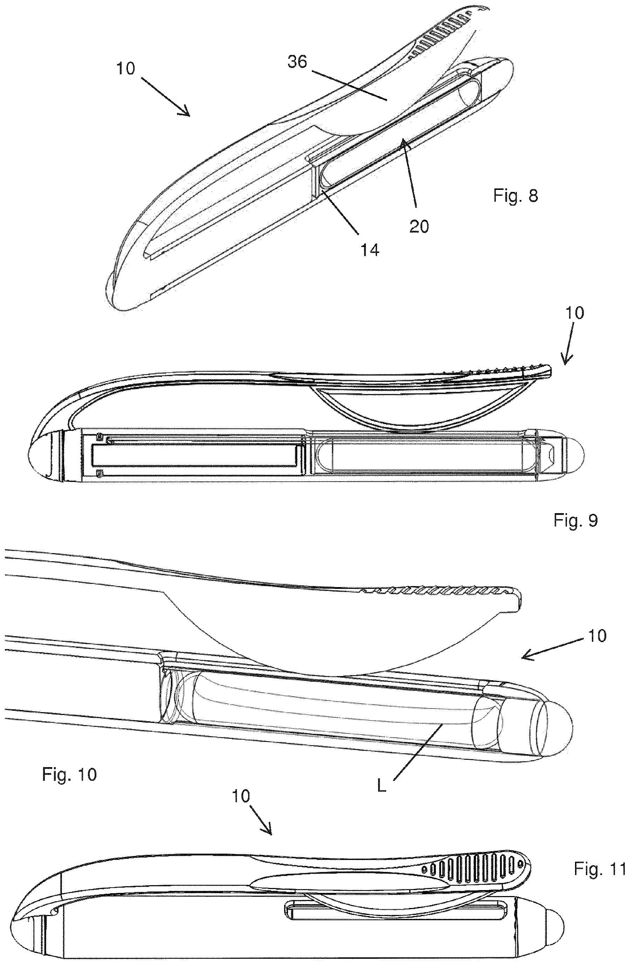

Figure 8 illustrates a sectioned perspective view of the liquid dispenser;
Figure 9 illustrates a sectioned side view of the liquid dispenser;
Figure 10 illustrates an enlarged sectioned view of a forward portion of the liquid dispenser;
Figure 11 illustrates an alternative perspective view of the liquid dispenser;
Figure 12 illustrates a side elevation of the liquid dispenser with an actuator in a disengaged position;
Figure 13 illustrated a sectioned elevation of the liquid dispenser as illustrated in Figure 12;
Figure 14 illustrates a side elevation of the liquid dispenser with the actuator in a partially engaged or depressed position;
Figure 15 illustrated a sectioned elevation of the liquid dispenser as illustrated in Figure 15;
Figure 16 illustrates a side elevation of the liquid dispenser with the actuator in a fully engaged or depressed position;
Figure 17 illustrates a sectioned elevation of the liquid dispenser as illustrated in Figure 16;
Figure 18 illustrates a side elevation of the liquid dispenser with the actuator fully depressed in order to dispense liquid;
Figure 19 illustrates a sectioned elevation of the liquid dispenser of Figure 18 with liquid being dispensed therefrom;
Figure 20 illustrates an end view of the arrangement of Figures 12 and 13;
Figure 21 illustrates a sectioned end view of the arrangement of Figures 12 and 13;
Figure 22 illustrates an end view of the arrangement of Figures 14 and 15;
Figure 23 illustrates a sectioned end view of the arrangement of Figures 14 and 15;
Figure 24 illustrates an end view of the arrangement of Figures 16 and 17;
Figure 25 illustrates a sectioned end view of the arrangement of Figures 16 and 17;
Figure 26 illustrates an end view of the arrangement of Figures 18 and 19;
Figure 27 illustrates a sectioned end view of the arrangement of Figures 18 and 19;
Figure 28 illustrates an enlarged view of Figure 27; and
Figure 29 illustrates an enlarged view of Figure 19.

### Detailed description of the drawings

Referring now to the accompanying drawings there is illustrated a liquid dispenser, generally indicated as 10, having particular but not exclusive application in the dispensing of a liquid adhesive such as a cyanoacrylate based adhesive for topical application in the closure of a wound. The liquid dispenser 10 may however be used for dispensing other liquids, gels or flowable materials.

The liquid dispenser 10 comprises a body 12 which in the embodiment illustrated is of hollow cylindrical form, such as to define a chamber 14 on an interior thereof. It will however be appreciated from the following description of the configuration and operation of the dispensing device 10 that the body 12 may take any other suitable shape, and may vary in dimension in order to define varying volumetric capacities and/or cross sectional shapes of the chamber 14 as will become apparent from the following description.

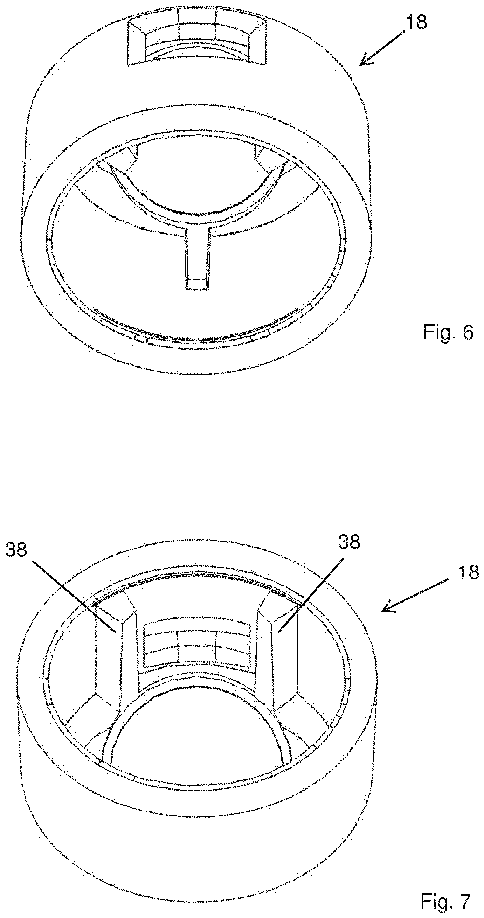

The body 12 additionally defines an outlet at one end thereof which permits fluid to be dispensed, in use, from the chamber 14 onto a wound or other application site. The opposed end of the body 12 is closed in use. In the embodiment illustrated the outlet is defined by a nozzle in the form of a dispensing tip 18 which retains a foam filter 40 as described hereinafter in order to permit the controlled dispensing of liquid from within the chamber 14. The tip 18 is shown in isolation in Figures 6 and 7, but is preferably formed integrally with the rest of the body 12. It is envisaged that the dispensing tip 18 may be replaced or augmented with alternative terminations such as a brush head, sponge, foam or filter type applicator, fine point nozzle or the like. In that case the dispensing tip 18 may be releasably retained on the body 12 by any suitable means, for example one or more resiliently deformable tabs or barbs, or threaded on to the body 12.

The liquid dispenser 10 is designed to receive and retain, preferably releasably, in the chamber 14, a single use container in the form of a cartridge 20, holding a discrete quantity of a liquid L to be dispensed, and as visible in the various sectioned views of the drawings. The body 12 and chamber 14 are thus preferably shaped and dimensioned to provide a complimentary fit such that the cartridge 20 is substantially immobilized within the chamber 14, although with sufficient space around the cartridge 20 to permit deformation when force is applied, as described in greater detail hereinafter. A number of longitudinally extending ribs 38 may be provided on the inner wall of the chamber 14 in order to further improve the fit between the cartridge 20 and chamber 14, while also providing the surrounding space necessary for deformation of the cartridge 20.

The cartridge 20 comprises a cylindrical ampoule 22 within which the discrete quantity of liquid L is contained, the ampoule 22 being at least partially formed from a brittle material that may be ruptured or breached through the application of force, for example glass, rigid plastic or similar material. The ampoule 22 is also preferably translucent in order to allow a user to ascertain the presence of the liquid L therein. The cartridge 20 further comprises an outer sleeve 24 closely surrounding and enveloping the ampoule 22, the outer sleeve 24 being formed from a flexible or otherwise resiliently deformable material, for example a flexible polymer based material or the like. Again the outer sleeve 24 is preferably translucent in order to enable a user to ascertain the presence of the liquid L within the ampoule 22. While the ampoule 22 is completely closed to the external environment, the outer sleeve 24 includes at least one open end 26. In this way, and as will be described hereinafter in detail, the ampoule 22 may be broken or otherwise ruptured in order to enable the liquid L to flow outwardly therefrom, and the liquid L can then be dispensed from the open end 26 of the outer sleeve 24. The flexible outer sleeve 24 allows a deforming force to be applied through the outer sleeve 24 to the rigid ampoule 22 which will break once a suitable force is applied thereto. As the outer sleeve 24 is flexible it will not rupture or otherwise break and will thus retain a liquid impermeable barrier surrounding the broken ampoule 22 which will thus ensure that the liquid L is only capable of flowing outwardly through the open end 26,via the filter 40 retained at the open end 26 and which thus allows the passage of the liquid L while preventing the escape of any of the broken pieces of the ampoule 22, which in most cases will be formed from glass. Although less preferred, it is also envisaged that the outer sleeve 24 could be omitted, and instead the outer sleeve 24 could be permanently or releasably located as an insert or lining within the chamber 14, into which the bare ampoule 22 could then be inserted.

Returning to the dispensing device 10, the body 12 further defines a port or window 28 which provides access to the chamber 14 and in the embodiment illustrated is in the form of an elongate slot running longitudinally to the body 12 to overlie the cartridge 20 when contained within the chamber 14 The dispensing device 10 further comprises an actuator in the form of a cantilever arm 30 projecting from adjacent one end of the body 12, the cantilever arm 30 having a fixed end 32 secured to or formed integrally with the body 12, and an opposed free end 34, the cantilever arm 30 extending substantially longitudinally of the body 12 and at least partially overlying the window 28. In the preferred embodiment illustrated the cantilever arm 30 is formed integrally with a holding shaft 30a that is shaped and dimensioned to be inserted into the end of the body 12 opposite the outlet 18 such as to close that end of the body 12 and to be permanently retained therein. The holding shaft 30a may define an interference fit with the interior of the body 12, and/or may be secured with an adhesive or the like, or by mechanical means to preferably provide an irreversible connection between the body 12 and the cantilever arm 30. For example the inner wall of the chamber 14 may be provided with one or more indents while the holding shaft 30a is provided with one or more corresponding protrusions. Once inserted into the body 12 the cantilever arm 30 and the body 12 effectively become a single integrated part. As an alternative to the above configuration the cantilever arm 30 could be formed integrally with the body 12, for example as a single molded, printed or otherwise formed component. These configurations are designed such that the liquid dispensing device 10 takes the form of a conventional pen which is thus familiar to the user and is preferably dimensioned to be comfortable and practical for manual manipulation.

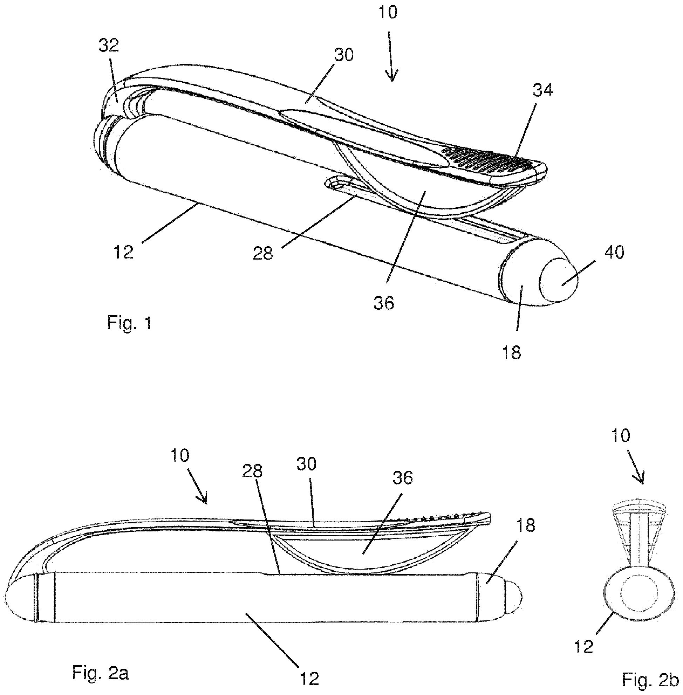

The cantilever arm 30 is displaceable relative to the body 12 through the application of manual force, most effectively about the free end 34 and in a direction radially inwardly of the body 12, this displacement preferably being facilitated by the use of a resiliently deformable material forming at least a part of the cantilever arm 30, although any other suitable means may be utilized in order to facilitate this displacement. In the preferred embodiment illustrated the cantilever arm 30 is reversibly displaceable relative to the body 12, the fixed end 32 defining a live hinge between the cantilever arm 30 and the holding shaft 30a or effectively the body 12. Thus when the manual force is released the cantilever arm 30 will return to the un-deformed position, for example as illustrated in Figures 1 and 2.

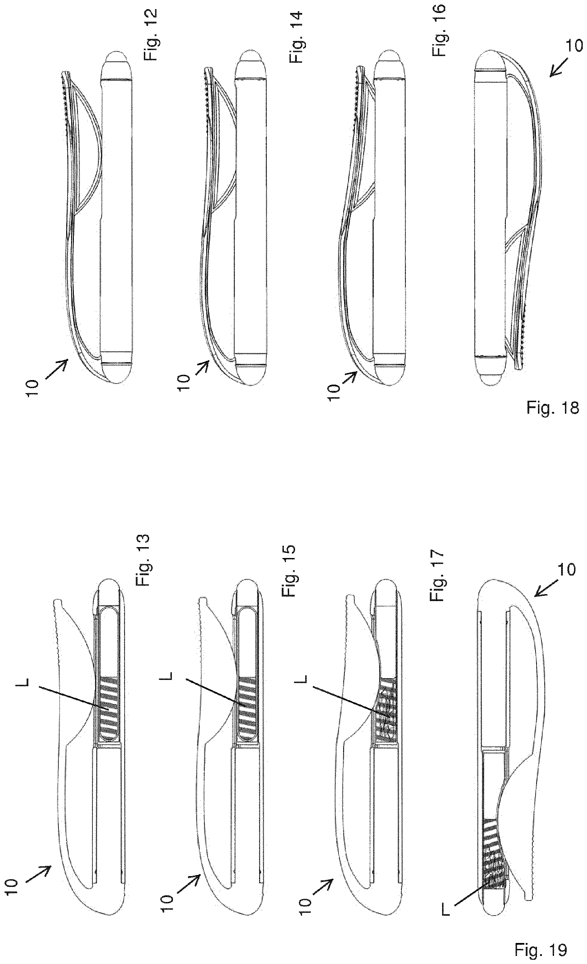

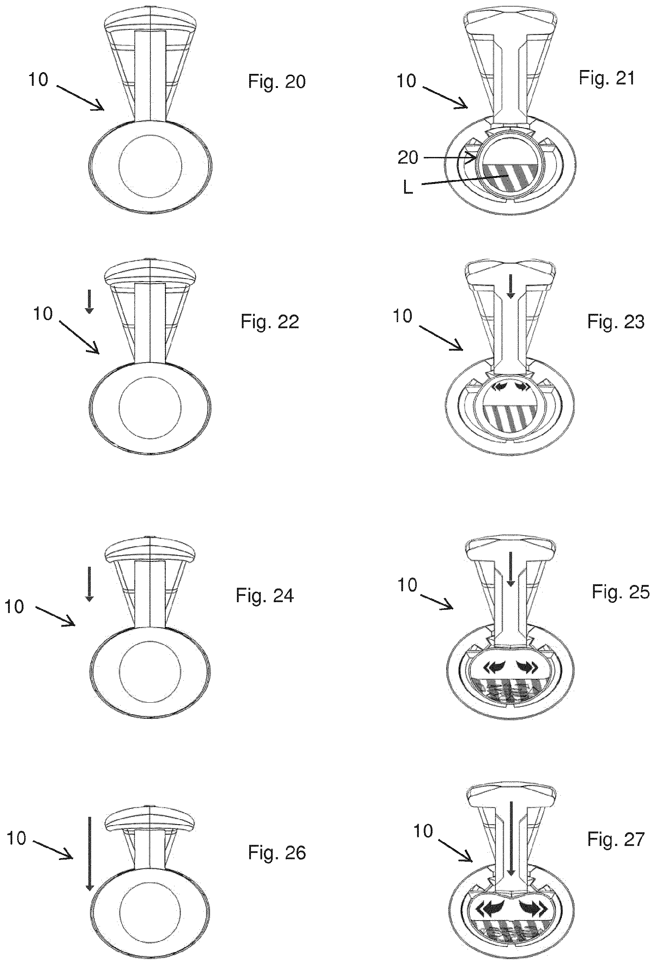

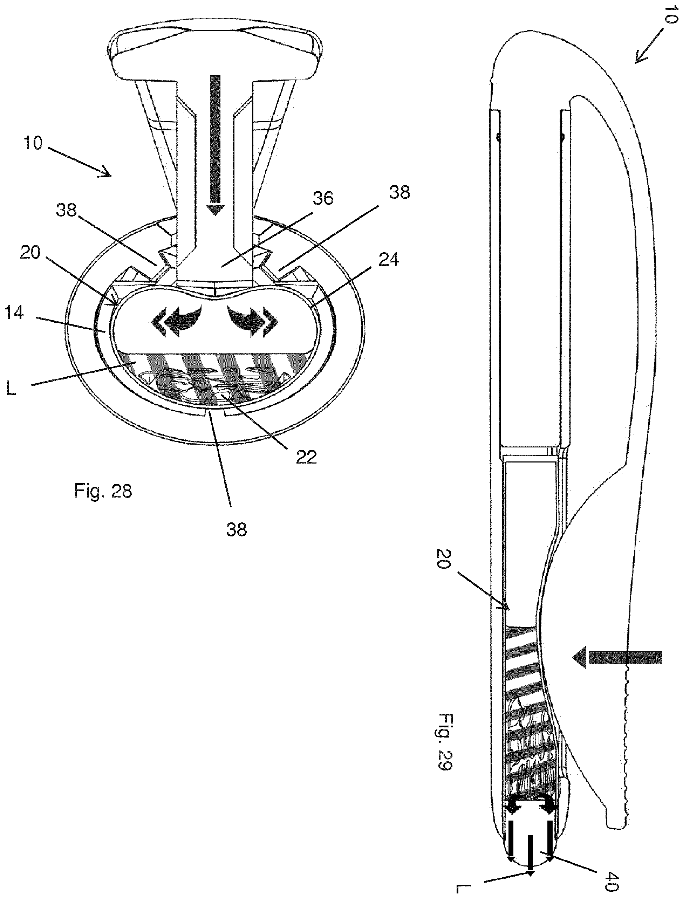

The cantilever arm 30 comprises an abutment 36 extending outwardly from the cantilever arm 30 towards the body 12, and preferably overlying a substantial portion of the window 28. The abutment 36 is dimensioned to sit outwardly of the chamber 14 within the body 12 when the cantilever arm 30 is in the un-deformed state, for example as illustrated in Figures 1 and 2, where no external force is being applied to the cantilever arm 30. The cantilever arm 30 and abutment 36 may however be arranged such that the distal edge of the abutment 36 sits within the window 28 but does not extend into the chamber 14, when no external force is being applied, in order to prevent any unintended lateral deflection of the cantilever arm 30 during the initial depression of the arm 30. The abutment 36 is however dimensioned, in particular in width, to be capable of passing through the window 28 into the chamber 14 when the cantilever arm 30 is deformed inwardly towards the body 12. Figures 14 and 15 illustrate the cantilever arm 30 in a partially depressed state showing the abutment 36 beginning to pass through the window 28. At this point it will be appreciated that the abutment 36 will contact the outer sleeve 24 of the cartridge 20, and the further application of force by an operator will begin to apply a force to the cartridge 20 thereby effectively clamping the cartridge 20 between the abutment 36 and the inner wall of the chamber 14 opposite the window 28. By applying for the force to the cantilever armed 30 the abutment 36 will rupture the ampoule 22, and in the case of a glass ampoule 22 will shatter the glass, thereby permitting the liquid L to flow from the ampoule 22 into the outer sleeve 24, as illustrated in Figures 19 and 27. The broken pieces of the ampoule 22 can also be more clearly seen in Figure 29, captured and retained within the outer sleeve 24 while the liquid L can be dispensed via the tip 18 The abutment 36 preferably comprises a curved profile such that the contact patch with the cartridge 20 is relatively small and thus the force transmitted to that point on the ampoule 22 will be magnified. In this way the actuator 30 will not be required to be displaced with significant force in order to break the ampoule 22, thereby ensuring ease of use of the liquid dispenser 10. In addition, the elongate form of the window 28 and the abutment 36 will ensure that lateral deflection of the actuator 30 does not occur during the application of force to the ampoule 22. Allowing the ventral surface of the abutment 36 to sit within the window 28 when no external force is being applied ensures that this lateral deflection is completely avoided, in particular at the beginning of the inward depression of the cantilever arm 30.

In use the cartridge 20 is oriented within the dispensing device 10 such that the open end 26 of the outer sleeve 24 is in register with the outlet as defined by the dispensing tip 18. Thus by rupturing the ampoule 22 the liquid L can flow through the outer sleeve 24, via the open end 26 to the dispensing tip 18 to be dispensed from the dispensing device 10 as required. By repeatedly releasing and depressing the cantilever arm 30 a user can then effectively pump the liquid L from the chamber 14 as required.

In order to prevent any parts of the broken ampoule 22 from being dispensed along with the liquid L the liquid dispenser 10 incorporates the filter 40 located between the chamber 14 and the dispensing tip 18, and in the embodiment illustrated is retained within the dispensing tip 18. The filter 40 is designed to permit the flow of the liquid L to exit from the dispensing tip 18, but to trap any pieces or particles of broken glass or the like from the ampoule 22.

Depending on the liquid L to be dispensed, it may be necessary to provide an initiator or similar catalyst which is adapted to effect a physical and/or chemical change in the liquid L such as to have the requisite properties for its intended application, for example to cure when used as a surgical adhesive. It is preferable that the initiator is maintained out of contact with the liquid L until the ampoule 22 is broken, at which point the liquid pouring from the ampoule 22 is brought into contact with the initiator. In the case of a cyanoacrylate adhesive the initiator is operable to effect the polymerization of the monomer to a polymer. It will of course be appreciated that any other suitable initiator/catalyst may be employed if necessary.

The use of the cantilever arm 30 in order to effect the rupturing of the ampoule 22 and the subsequent pumping of the liquid L from the dispensing device 10 enables, through the use of leverage, significant force to be applied by a user in order to break the ampoule 22, and then allows substantial modulation of the pumping of the liquid L from the dispensing device 10, thereby allowing a greater degree of control in metering the flow of liquid L. In addition the use of the cantilever arm 30 to displace the abutment 36 through the window 28 enables the body 12 to be formed from a rigid material whilst still allowing controlled access to the cartridge 20 contained within the chamber 14, thereby essentially eliminating the possibility of a shard of broken glass from the ampoule 22 piercing the body 12 and potentially causing harm to the operator, were such a piece of glass to pierce the outer sleeve 24.

In order to improve the operation of the dispensing device 10, in particular to reduce the force required to depress the cantilever arm 30 against the cartridge 20 such as to rupture the ampoule 22, the ampoule 22 and the chamber 14 are preferably designed such that the chamber 14 has a cross sectional area greater than the cross sectional area of the ampoule 22/cartridge 20 such as to allow deformation of the cartridge 20 when the crushing force is applied by the abutment 36 of the cantilever arm 30. In the preferred embodiment illustrated this is achieved through the use of an ampoule 22 which has a circular cross section, as is conventional for such commercially available ampoules, while the chamber 14 is designed with an oval or elliptical cross sectional area whose minor axis accommodates the diameter of the cartridge 20 and whose major axis is greater than the diameter of the cartridge 20. This creates empty space around the unstressed cartridge 20 which thus allows the cartridge 20 to deform outwardly when the crushing force is applied, thereby allowing the walls of the ampoule 22 to deform and ultimately break, permitting the liquid contained therein to be dispensed. This is most clearly illustrated in Figures 25, 26 and 28. The longitudinally extending ribs 38 ensure that the cartridge 20 containing the ampoule 22 is substantially immobilised within the chamber 14 despite the provision of space around the cartridge 20 to facilitate the above mentioned extensive and controlled deformation. Without this difference in cross sectional areas, the cartridge 20 can not deform when pressure is applied by the abutment 36, and the walls of the chamber 14 therefore act like a reinforcing ring around the ampoules, significantly increased the force necessary to rupture the ampoule 22.

It will be appreciated that while the preferred embodiment of the dispensing device 10 of the present invention employs an oval cross section for the chamber 14, any other functional alternative may be employed which provides space between the unstressed cartridge 20 and the walls of the chamber 14, into which space the ampoule 22 can undergo extensive and controlled deformation to the point that the wall of the ampoule deforms beyond the elastic limit of the particular material, leading to cracking of the wall of the ampoule 22. Essentially this functionality is achieved through a mismatch in the volume or cross sectional area between the chamber 14 and the ampoule 22.

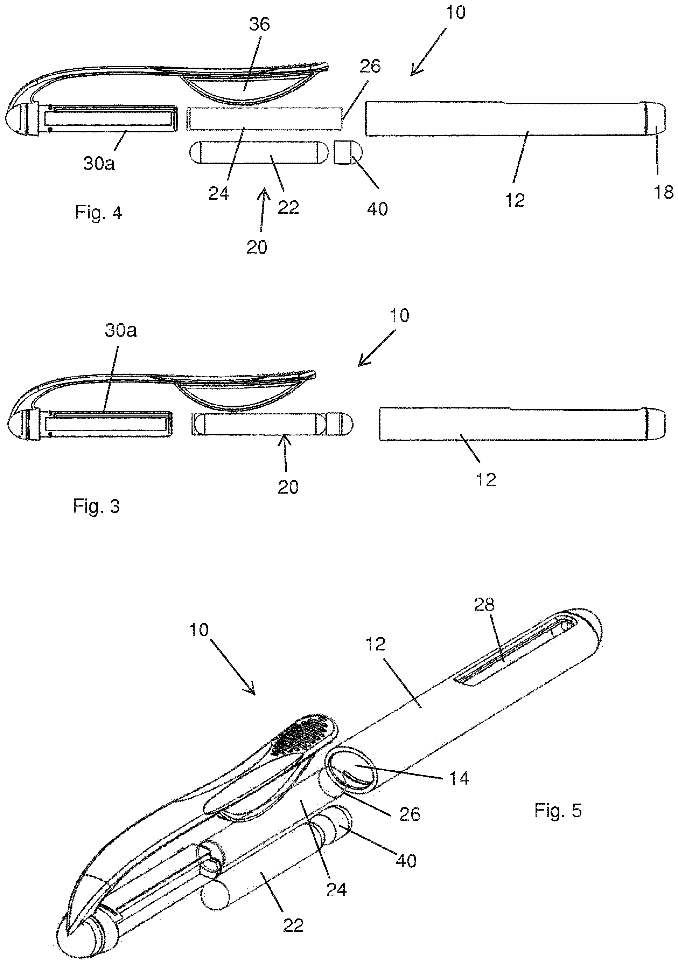

The dispensing device 10 may be a reusable device which allows an expired cartridge 20 or ampoule 22 to be removed from the chamber 14 and replaced. The dispensing tip 18 and/or the plug 30a may therefore be releasably retained on the body 12 by suitable means, and when removed provides access to the chamber 14 in order to allow the cartridge 20 or ampoule 22 to be inserted and removed as necessary. The dispensing tip 18, in particular when incorporating the filter 40, may be provided as a consumable item to be replaced along with the cartridge 20. Thus the ampoule 22, and filter 40 may be provided as a combined unit, in the form of the cartridge 20 as illustrated in Figure 3, whereby the filter 40 is captured within the open end 26 of the outer sleeve 24 which open end 26 may then be captured within the dispensing tip 18.

The liquid dispenser 10 of the present invention therefore provides a simple, ergonomic and functional means of dispensing liquids in safe, reliable and controlled manner.

## Claims

1. A liquid dispenser (10) comprising an ampoule (22) of a liquid to be dispensed; a housing (12) defining a chamber (14) for receiving the ampoule, the housing further defining an outlet to enable the liquid to be dispensed from the chamber; a flexible outer sleeve (24) surrounding the ampoule and having at least one open end (26); an actuator (30) displaceable relative to the housing; a section (28) of the housing (12) being adapted to permit a least a section of the actuator (30) to be displaced into the chamber (14) to apply a force, in use, to the ampoule (22); and wherein the chamber (14) has a cross sectional area greater than the cross sectional area of the ampoule (22) and dimensioned such as to allow deformation of the ampoule (22) when the force is applied; **characterised in that** the chamber has an elliptical cross sectional area.

2. A liquid dispenser according to claim 1 in which the housing (12) comprising one or more elongate ribs (38) extending longitudinally of the chamber.

3. A liquid dispenser according to claim 1 or 2 in which the section of the housing (12) permitting access to the chamber (14) is defined by a window (28) in the housing.

4. A liquid dispenser according to claim 3 in which the actuator (30) comprises an abutment (36) shaped and dimension to pass through the window (28).

5. A liquid dispenser according to claim 3 or 4 in which the actuator (30) comprises a cantilever arm (30) projecting from the housing (12).

6. A liquid dispenser according to claim 5 in which the abutment (36) is located at or adjacent to a free end of the cantilever arm (30).

7. A liquid dispenser according to any preceding claim in which the actuator (30) is reversibly displaceable relative to the housing (12).

8. A liquid dispenser according to any preceding claim in which the actuator (30) is hingedly mounted to the housing (12).

9. A liquid dispenser according to any of claims 5 to 8 in which the actuator (30) is biased away from the housing (12).

10. A liquid dispenser according to any of claims 5 to 9 in which at least a portion of the actuator (30) is formed from a resiliently deformable material.

11. A liquid dispenser according to any preceding claim in which the outlet comprises a nozzle (18) in fluid communication with the chamber.

12. A liquid dispenser according to claim 11 in which the nozzle (18) is releasably retained on the housing (12) in order to permit access to the chamber (14).

13. A liquid dispenser according to any preceding claim in which the housing (12) comprises a substantially cylindrical hollow body.

14. A liquid dispenser according to any preceding claim in which the housing (12) is rigid.

15. A liquid dispenser according to any preceding claim in which the ampoule comprises a breachable wall.

## Patentansprüche

1. Flüssigkeitsspender (10), umfassend eine Ampulle (22) einer abzugebenden Flüssigkeit; ein Gehäuse (12), das eine Kammer (14) zum Aufnehmen der Ampulle definiert, wobei das Gehäuse ferner einen Auslass definiert, damit die Flüssigkeit aus der Kammer abgegeben werden kann; eine flexible Außenhülse (24), welche die Ampulle umgibt und mindestens ein offenes Ende (26) aufweist; ein Betätigungselement (30), das relativ zu dem Gehäuse verschiebbar ist; wobei ein Abschnitt (28) des Gehäuses (12) gestaltet ist, zu ermöglichen, dass mindestens ein Abschnitt des Betätigungselements (30) in die Kammer (14) verschoben werden kann, um bei Verwendung eine Kraft auf die Ampulle (22) aufzubringen; und wobei die Kammer (14) eine Querschnittsfläche aufweist, die größer als die Querschnittsfläche der Ampulle (22) und so bemessen ist, um eine Verformung der Ampulle (22) beim Aufbringen der Kraft zu ermöglichen; **dadurch gekennzeichnet, dass** die Kammer eine elliptische Querschnittsfläche aufweist.

2. Flüssigkeitsspender nach Anspruch 1, bei dem das Gehäuse (12) eine oder mehrere längliche Rippen (38) umfasst, die sich in Längsrichtung der Kammer erstreckt/erstrecken.

3. Flüssigkeitsspender nach Anspruch 1 oder 2, bei dem der Abschnitt des Gehäuses (12), der einen Zugang zu der Kammer (14) gestattet, durch ein Fenster (28) in dem Gehäuse definiert ist.

4. Flüssigkeitsspender nach Anspruch 3, bei dem das Betätigungselement (30) einen Anschlag (36) umfasst, der geformt und bemessen ist, um durch das Fenster (28) hindurchzupassen.

5. Flüssigkeitsspender nach Anspruch 3 oder 4, bei dem das Betätigungselement (30) einen Auslegerarm (30) umfasst, der von dem Gehäuse (12) vorsteht.

6. Flüssigkeitsspender nach Anspruch 5, bei dem sich der Anschlag (36) an einem freien Ende oder angrenzend an ein freies Ende des Auslegerarms (30) befindet.

7. Flüssigkeitsspender nach einem vorhergehenden Anspruch, bei dem das Betätigungselement (30) gegenüber dem Gehäuse (12) reversibel verschiebbar ist.

8. Flüssigkeitsspender nach einem vorhergehenden Anspruch, bei dem das Betätigungselement (30) an dem Gehäuse (12) angelenkt ist.

9. Flüssigkeitsspender nach einem der Ansprüche 5 bis 8, bei dem das Betätigungselement (30) weg von dem Gehäuse (12) vorgespannt ist.

10. Flüssigkeitsspender nach einem der Ansprüche 5 bis 9, bei dem zumindest ein Teil des Betätigungselements (30) aus einem elastisch verformbaren Material gebildet ist.

11. Flüssigkeitsspender nach einem vorhergehenden Anspruch, bei dem der Auslass eine Düse (18) in Fluidverbindung mit der Kammer umfasst.

12. Flüssigkeitsspender nach Anspruch 11, bei dem die Düse (18) lösbar an dem Gehäuse (12) gehalten wird, um einen Zugang zu der Kammer (14) zu ermöglichen.

13. Flüssigkeitsspender nach einem vorhergehenden Anspruch, bei dem das Gehäuse (12) einen im Wesentlichen zylindrischen Hohlkörper umfasst.

14. Flüssigkeitsspender nach einem vorhergehenden Anspruch, bei dem das Gehäuse (12) starr ist.

15. Flüssigkeitsspender nach einem vorhergehenden Anspruch, bei dem die Ampulle eine zerbrechbare Wand umfasst.

## Revendications

1. Distributeur de liquide (10) comprenant une ampoule (22) d'un liquide à distribuer ; un boîtier (12) définissant une chambre (14) pour recevoir l'ampoule, le boîtier définissant en outre une sortie pour permettre au liquide d'être distribué depuis la chambre ; un manchon externe flexible (24) encerclant l'ampoule et ayant au moins une extrémité ouverte (26) ; un actionneur (30) déplaçable par rapport au boîtier ; une section (28) du boîtier (12) étant adaptée pour permettre à au moins une section de l'actionneur (30) d'être déplacée dans la chambre (14) pour appliquer une force, en utilisation, à l'ampoule (22) ;
et dans lequel la chambre (14) présente une aire en coupe transversale supérieure à l'aire en coupe transversale de l'ampoule (22) et dimensionnée de sorte à permettre une déformation de l'ampoule (22) lorsque la force est appliquée ; **caractérisé en ce que** la chambre présente une aire en coupe transversale elliptique.

2. Distributeur de liquide selon la revendication 1, dans lequel le boîtier (12) comprend une ou plusieurs nervures allongées (38) s'étendant longitudinalement par rapport à la chambre.

3. Distributeur de liquide selon la revendication 1 ou 2, dans lequel la section du boîtier (12) permettant un accès à la chambre (14) est définie par une fenêtre (28) dans le boîtier.

4. Distributeur de liquide selon la revendication 3, dans lequel l'actionneur (30) comprend une butée (36) formée et dimensionnée pour passer à travers la fenêtre (28).

5. Distributeur de liquide selon la revendication 3 ou 4, dans lequel l'actionneur (30) comprend un bras en porte-à-faux (30) en saillie depuis le boîtier (12).

6. Distributeur de liquide selon la revendication 5, dans lequel la butée (36) est située à une extrémité libre du bras en porte-à-faux (30) ou adjacente à celle-ci.

7. Distributeur de liquide selon une quelconque revendication précédente, dans lequel l'actionneur (30) est déplaçable de manière réversible par rapport au boîtier (12).

8. Distributeur de liquide selon une quelconque revendication précédente, dans lequel l'actionneur (30) est monté de manière articulée sur le boîtier (12).

9. Distributeur de liquide selon l'une quelconque des revendications 5 à 8, dans lequel l'actionneur (30) est sollicité en éloignement du boîtier (12).

10. Distributeur de liquide selon l'une quelconque des revendications 5 à 9, dans lequel au moins une portion de l'actionneur (30) est formée à partir d'un matériau déformable de manière résiliente.

11. Distributeur de liquide selon une quelconque revendication précédente, dans lequel la sortie comprend une buse (18) en communication fluidique avec la chambre.

12. Distributeur de liquide selon la revendication 11, dans lequel la buse (18) est retenue de manière libérable sur le boîtier (12) afin de permettre un accès à la chambre (14).

13. Distributeur de liquide selon une quelconque revendication précédente, dans lequel le boîtier (12) comprend un corps creux sensiblement cylindrique.

14. Distributeur de liquide selon une quelconque revendication précédente, dans lequel le boîtier (12) est rigide.

15. Distributeur de liquide selon une quelconque revendication précédente, dans lequel l'ampoule comprend une paroi fracturable.
